(19) Europäisches Patentamt
European Patent Office
Office européen des brevets

(11) Veröffentlichungsnummer: **0 092 517**
**B1**

(12) # EUROPÄISCHE PATENTSCHRIFT

(45) Veröffentlichungstag der Patentschrift: **12.03.86**

(51) Int. Cl.⁴: **C 07 D 317/22,** C 07 D 317/72, A 01 N 43/28

(21) Anmeldenummer: **83810144.2**

(22) Anmeldetag: **11.04.83**

(54) **Neue Oximäther, Verfahren zu ihrer Herstellung, Mittel die die neuen Oximäther enthalten und ihre Verwendung.**

(30) Priorität: **16.04.82 CH 2326/82**

(43) Veröffentlichungstag der Anmeldung: **26.10.83 Patentblatt 83/43**

(45) Bekanntmachung des Hinweises auf die Patenterteilung: **12.03.86 Patentblatt 86/11**

(84) Benannte Vertragsstaaten: **BE CH DE FR GB IT LI NL**

(56) Entgegenhaltungen: **EP - A - 0 011 047**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Martin, Henry, Dr., Jupiterstrasse 17, CH-4123 Allschwil (CH)**
Erfinder: **Fricker, Urs, Baumgartenweg 8, CH-4460 Gelterkinden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue Oximäther, Verfahren zu ihrer Herstellung, Mittel zum Schutz von Kulturpflanzen vor der phytotoxischen Wirkung von Herbiziden, die die neuen Oximäther als aktive Komponente enthalten und ihre Verwendung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäuren usw. bei der Anwendung in wirksamer Dosis zuweilen neben den zu bekämpfenden Unkräutern auch die Kulturpflanzen in gewissem Masse schädigen. Um diese Problem zu begegnen, sind schon verschiedene Stoffe vorgeschlagen worden, welche befähigt sind, die schädigende Wirkung des Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

So beschreibt die Britische Patentschrift Nr. 1 277 557 die Behandlung von Samen bzw. Sprösslingen von Weizen und Sorghum mit gewissen Oxamsäureestern und Amiden zum Schutz vor dem Angriff durch «ALACHLOR» (N-Methoxymethyl-N--chloracetyl-2,6-diäthylanilin). In den Deutschen Offenlegungsschriften 1 952 910 und 2 245 471, sowie in der Französischen Patentschrift 2 021 611 werden Gegenmittel zur Behandlung von Getreide-, Mais- und Reissamen zum Schutz gegen die schädigende Einwirkung von herbizid wirksamen Thiolcarbamaten vorgeschlagen. Gemäss der Deutschen Patentschrift 1 576 676 und der US-Patentschrift 3 131 509 werden Hydroxyaminoacetanilide und Hydantoine für den Schutz von Getreidesamen gegenüber Carbamaten verwendet.

Die direkte pre- oder postermergente Behandlung gewisser Nutzpflanzen mit Gegenmitteln als Antagonisten bestimmter Herbizidklassen auf einer Anbaufläche ist in den Deutschen Offenlegungsschriften 2 141 586 und 2 218 097, sowie im US-Patent 3 867 444 beschrieben.

Ferner können Maispflanzen gemäss der Deutschen Offenlegungsschrift 2 402 983 wirksam vor Schädigung durch Chloracetanilide geschützt werden, indem man dem Boden als Gegenmittel ein N-disubstituiertes Dichloracetamid zuführt.

Gemäss EP-A 11 047 können auch Alkoximinobenzylcyanide, deren Alkoxygruppe u.a. durch eine acetalisierte Carbonylgruppe substituiert ist, als Mittel zum Schutz von Kulturpflanzen von der schädigenden Wirkung von Herbiziden verschiedener Stoffklassen verwendet werden.

Gemäss vorliegender Erfindung werden neue Oximäther der Formel I

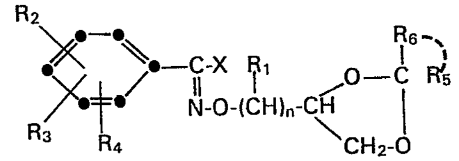

(I)

in welcher n 1 oder 2, $R_1$ und $R_2$ je Wasserstoff oder $C_1$-$C_4$ Alkyl, $R_3$ und $R_4$ je Wasserstoff, Halogen, $C_1$-$C_4$ Alkyl, $C_1$-$C_4$ Halogenalkyl, $C_1$-$C_4$ Alkoxy, $C_1$-$C_4$ Halogenalkoxy, $C_1$-$C_4$ Alkylthio, $C_1$-$C_4$ Halogenalkylthio, $C_1$-$C_4$ Alkylsulfinyl, $C_1$-$C_4$ Alkylsulfonyl, $C_1$-$C_4$ Halogenalkylsulfinyl, $C_1$-$C_4$ Halogenalkylsulfonyl oder Nitro, $R_5$ und $R_6$ einzeln je Wasserstoff, $C_1$-$C_4$ Alkyl, $C_1$-$C_4$ Halogenalkyl, Phenyl, unsubstituiert oder substituiert durch Halogen, $C_1$-$C_4$ Alkyl, $C_1$-$C_4$ Halogenalkyl, $C_1$-$C_4$ Alkoxy, $C_1$-$C_4$ Halogenalkoxy, $C_1$-$C_4$ Alkylthio, $C_1$-$C_4$ Alkylsulfinyl, $C_1$-$C_4$ Alkylsulfonyl, Carboxyl, Carbamoyl, $C_1$-$C_4$-Alkylcarbamoyl, Nitro oder Cyan, $R_5$ und $R_6$ zusammen auch eine 2 bis 6gliedrige Alkylen- oder Alkenylenkette, die durch $C_1$-$C_4$ Alkylreste substituiert sein kann, X Wasserstoff, Cyan, Nitro, Chlor, $C_1$-$C_4$ Alkyl, $C_1$-$C_4$ Halogenalkyl, $C_3$-$C_6$ Cycloalkyl, Carboxyl, Carbamoyl, $C_1$-$C_4$ Alkylcarbonyl, $C_1$-$C_4$ Alkoxycarbonyl, $C_1$-$C_4$ Alkylcarbamoyl bedeuten, vorgeschlagen.

Die als bzw. in den Resten $R_1$-$R_6$ vorkommenden $C_1$-$C_4$ Alkylgruppen können geradkettig oder verzweigt sein und bedeuten im einzelnen Methyl, Äthyl, n-Propyl, Isopropyl, n-Butyl, sek.-Butyl, Isobutyl und tert.-Butyl- Unter diesen Alkylgruppen sind $C_1$-$C_2$ Alkylgruppen bevorzugt und $R_1$ bedeutet als Alkylrest vorzugsweise Methyl.

Unter Halogen sind Fluor, Chlor, Brom und Jod, insbesondere aber Fluor und Chlor zu verstehen.

Der Halogenalkylrest X kann beispielsweise Difluormethyl, Trifluormethyl, Chlordifluormethyl, Tetrafluoräthyl, Pentafluoräthyl, und Heptafluorpropyl bedeuten. Besonders bevorzugte Reste X sind Trifluormethyl und Cyan.

Die Verbindungen der Formel I, in welchen n die Zahl 1 und $R_1$ Wasserstoff oder Methyl, $R_2$ Wasserstoff, $R_4$ Wasserstoff oder Halogen bedeuten, während $R_3$, $R_5$, $R_6$ und X die unter Formel I gegebene Bedeutung haben, sind bevorzugt. Sie entsprechen der Formel Ia

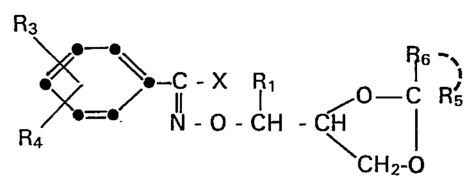

(Ia)

Von diesen erwiesen sich diejenigen Verbindungen als besonders aktiv, welche den Formeln Ib oder Ic entsprechen

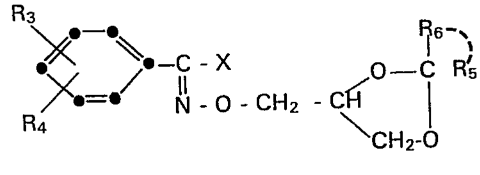

(Ib)

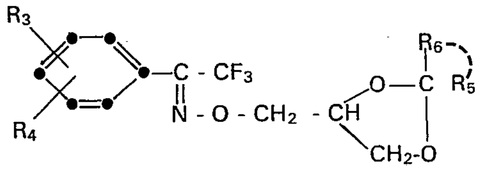
(Ic)

in welchen $R_3$, $R_5$ und $R_6$ die unter Formel I gegebene Bedeutung haben, während $R_4$ Wasserstoff oder Halogen bedeutet.

Bevorzugte Einzelverbindungen sind:

N-(1,3-Dioxolan-5-yl-methoxy)-Imino-para-chlorphenylacetonitril,

N-(4-Methyl-1,3-dioxolan-5-yl-methyl)-imino-para--chlorphenylacetonitril,

1-Phenyl-1-(1,3-dioxolan-5-yl-methoxyimino)-2,2,2--trifluoräthan,

1-(4-Chlorphenyl-1-(1,3-dioxolan-5-yl-methoxyimino)-2,2,2-trifluoräthan,

N-(2,2-Dimethyl-1,3-dioxolan-5-yl-methoxy)-imino--para-chlorphenylacetonitril,

N-(1,3-Dioxolan-5-yl-methoxy)-imino-phenylacetonitril,

N-(2-Methyl-1,3-dioxolan-5-yl-methoxy)-imino--ortho-fluorphenyl-acetonitril,

N-(2-Methyl-1,3-dioxolan-5-yl-methoxy)-imino-para--tolyl-acetonitril,

N-(2,2-Dimethyl-1,3-dioxolan-5-yl-methoxy)-imino--para-tolyl-acetonitril,

N-(1,3-Dioxolan-5-yl-methoxy)-imino-para-tolyl-acetonitril.

Die neuen Oximäther der Formel I werden erfindungsgemäss hergestellt, indem man ein Salz eines Oxims der Formel II

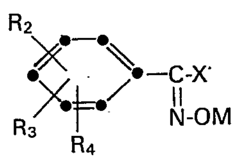
(II)

in welcher M ein Alkalimetall- oder Erdalkalimetallkation darstellt, und $R_2$, $R_3$, $R_4$ und X die oben angegebene Bedeutung haben, mit einem 5-(α-Halogenalkyl oder β-Halogenalkyl)-1,3-dioxolan der Formel III

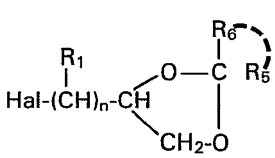
(III)

in welcher Hal ein Halogenatom, insbesondere ein Chloratom oder ein Bromatom, bedeutet und n, $R_1$, $R_5$ und $R_6$ die oben angegebene Bedeutung haben, umsetzt. Als Salze eines Oxims der Formel II sind insbesondere die Natrium- und Kaliumsalze geeignet. Die Umsetzung des Oxims der Formel II mit dem Halogenacetal der Formel III wird vorteilhaft in einem inerten organischen Lösungsmittel durchgeführt. Besonders geeignet sind polare Lösungsmittel, wie Acetonitril, Dimethylformamid und Dimethylsulfoxid. Die Reaktanten werden in der Regel in äquimolarer Menge eingesetzt. Es kann jedoch auch zum vollständigen Ablauf der Reaktion der eine oder der andere Reaktionspartner im Überschuss eingesetzt werden.

Die Umsetzung wird vorteilhaft bei erhöhter Temperatur, vorzugsweise bei Rückflusstemperatur des Reaktionsgemisches durchgeführt.

Die Oxime der Formel II, in denen X Halogenalkyl bedeutet, können in bekannter Weise durch Umsetzung der entsprechenden Ketone mit Hydroxylamin hergestellt werden. Die hierfür benötigten Ketone können ihrerseits durch Umsetzung einer Grignard-Verbindung der Formel IV

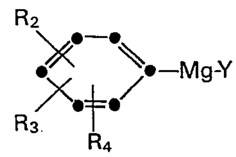
(IV)

in welcher Y Chlor, Brom oder Jod bedeutet und $R_2$, $R_3$ und $R_4$ die oben angegebene Bedeutung haben, mit einer Carbonsäure X'-COOH, einem Säurechlorid X'-COCl oder einem Nitril, worin X' Halogenalkyl bedeutet, erhalten werden (vgl. US-Patent 3 748 361).

Die Oximäther der Formel II, in denen X Cyan bedeutet, sind bekannte Verbindungen (siehe z.B. die US Patentschrift 3 799 757) oder können nach ähnlichen Methoden aus Benzylcyaniden, Alkylnitrit in Gegenwart von Natriumäthylat hergestellt werden [Organic Reactions (1953) Band 7, Seiten 343 und 373, Europäisches Patent 6152].

Oxime der Formel II, die zur Herstellung der neuen Oximäther der Formel I geeignet sind, sind beispielsweise:

1-Phenyl-1-hydroximino-2,2,2-trifluoräthan

1-(4-Methylphenyl)-1-hydroximino-2,2,2-trifluoräthan

1-(4-Chlorphenyl)-1-hydroximino-2,2,2-trifluoräthan

1-(4-Fluorphenyl)-1-hydroximino-2,2,2-trifluoräthan

1-(4-Trifluormethylphenyl)-1-hydroximino-2,2,2-trifluoräthan

1-(3-Trifluormethylphenyl)-1-hydroximino-2,2,2-trifluoräthan

1-(4-Methoxyphenyl)-1-hydroximino-2,2,2-trifluoräthan

1-(4-Trifluormethoxyphenyl)-1-hydroximino-2,2,2--trifluoräthan

1-(3-Nitrophenyl)-1-hydroximino-2,2,2-trifluoräthan

2-(4-Chlorphenyl)-2-hydroximino-acetonitril

2-(3-Chlorphenyl)-2-hydroximino-acetonitril

2-(2-Fluorphenyl)-2-hydroximino-acetonitril

2-(2,4-Dichlorphenyl)-2-hydroximino-acetonitril

2-(4-Fluorphenyl)-2-hydroximino-acetonitril

2-(2-Methylphenyl)-2hydroximino-acetonitril

2-(Phenyl)-2-hydroximino-acetonitril.

Die 5-Halogen-1,3-dioxolane, respektive 5-(α-Halogenalkyl)-1,3-dioxolane der Formel III können hergestellt werden, indem man halogeniertes Propylenoxid respektive einen 3-Halogenalkyl-1,2-diol der Formel VI

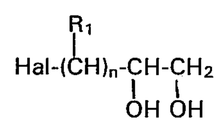
VI)

in welcher Hal für ein Halogenatom steht und $R_1$ die unter Formel I gegebene Bedeutung hat, mit einem niederen Keton oder Aldehyd der Formel VII

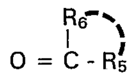 (VII)

in welcher $R_5$ und $R_6$ die unter Formel I gegebene Bedeutung haben, umsetzt.

Geeignete 5-Halogen- respektive 5-α-Halogenalkyl-1,3-dioxolane sind beispielsweise:
1-(1,3-Dioxolan-5-yl)-1-chloräthan
5-Chlormethyl-1,3-dioxolan
5-Brommethyl-1,3-dioxolan
5-Chlormethyl-2,2-dimethyl-1,3-dioxolan
5-Chlormethyl-2-äthyl-1,3-dioxolan
5-Brommethyl-2-methyl-1,3-dioxolan
5-Brommethyl-2-isopropyl-1,3-dioxolan
5-Chlormethyl-2-isobutyl-1,3-dioxolan
5-Brommethyl-2-tert.-butyl-1,3-dioxolan
5-Brommethyl-2,2-dimethyl-1,3-dioxolan
5-Brommethyl-2,2-diäthyl-1,3-dioxolan
5-(1,3-Dioxolan-5-yl)-1-bromäthan
5-(2-Methyl-1,3-dioxolan-5-yl)-1-bromäthan
5-(2,2-Dimethyl-1,3-dioxolan-5-yl)-1-bromäthan
5-(2-Äthyl-1,3-dioxolan-5-yl)-1-bromäthan
5-(5-Isopropyl-1,3-dioxolan-5-yl)-1-chloräthan
5-(2-Äthyl-2-methyl-1,3-dioxolan-5-yl)-1-chloräthan
5-Chlormethyl-2-äthyl-2-methyl-1,3-dioxolan
5-Chlormethyl-2,2-cyclopentyliden-1,3-dioxolan
5-Chlormethyl-2,2-cyclo-hexyliden-1,3-dioxolan.

Die neuen Oximäther der Formel I besitzen in hervorragendem Masse die Eigenschaft, Kulturpflanzen vor Schädigung durch Agrarchemikalien zu schützen. Dieser Schutz erstreckt sich insbesondere auf Herbizide der verschiedenen Stoffklassen, darunter 1,3,5-Triazine, 1,2,4-Triazinone, Phenylharnstoffderivate, Carbamate, Thiolcarbamate, Phenoxyessigsäureester, Phenoxypropionsäureester, Halogenacetanilide, Halogenphenoxyessigsäureester, substituierte Phenoxyphenoxyessigsäureester und -propionsäureester, substituierte Pyridyloxyphenoxyessigsäureester und -propionsäureester, Benzoesäurederivate usw., sofern diese nicht oder ungenügend kulturtolerant sind. Die neuen Oximäther der Formel I sind vor allem zum Schützen von Kulturpflanzen gegen die schädigende Wirkung von Halogenacetaniliden und Thiolcarbamaten geeignet. Die Oximäther der Formel I können daher in bezug auf ihre Anwendung in Kombination mit den vorgenannten Herbiziden als Gegenmittel oder «Antidotes» oder auch als «Safener» bezeichnet werden.

Von den Verbindungen der Formel I existieren verschiedene isomere Formen. Als Oximderivate liegen diese Verbindungen in der Syn- bzw. Anti-Form, bzw. als deren Gemische vor [E- und Z-Form s.R.S. Cohn et al. Ang. Chemie Int. Ed. 5 (1966) 385 oder Experientia 12 (1956) p. 81].

Je nach der Substitution der Verbindungen der Formel I besteht ein asymetrisches C-Atom und es können zwei enantiomere Formen vorliegen. Im allgemeinen entsteht bei der Herstellung ein Gemisch beider Enantiomeren welches sich auf übliche Weise in die optischen Antipoden auftrennen lässt.

Die monosubstituierte Dioxolane führen zu weiteren cis-trans Isomeren bzw. zu deren Gemischen. Zweifachsubstituierte Dioxolane führen zu cis-Syn und zu cis-Anti sowie trans Isomeren bzw. deren Gemischen. Durch die Auswahl der Ausgangsmaterialien, beispielsweise durch Verwendung reiner Z-Form der Oxime lassen sich die Isomeren vermindern. Diese verschiedenen Isomeren sowie deren Gemische sind auch Gegenstand der Erfindung.

Ein solches Gegenmittel oder Antidote der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die preemergente Behandlung schliesst sowohl die Behandlung der Anbaufläche vor der Aussaat (ppi = pre plant incorporation) als auch die Behandlung der angesägten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1 : 100 bis 5 : 1. In der Regel wird bei einem Mengenverhältnis von Gegenmittel zu Herbizid von 1 : 1 bis 1 : 20 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,1 - 10 g Gegenmittel benötigt. In der Regel wird mit 0,1 - 2 g Gegenmittel pro kg Samen bereits die volle Schutzwirkung erreicht. Falls das Gegenmittel kurz vor der Aussaat durch Samenquellung appliziert werden soll, so werden zweckmässig Lösungen des Gegenmittels, welche den Wirkstoff in einer Konzentration von 1 - 10'000 ppm enthalten. In der Regel wird mit Konzentrationen des Gegenmittels von 10 - 1000 ppm die volle Schutzwirkung erreicht.

In der Regel liegt zwischen protektiven Massnahmen, wie Samenbeizung und Behandlung von Stecklingen mit einem Gegenmittel der Formel I und der möglichen späteren Feldbehandlung mit Agrarchemikalien ein längerer Zeitraum, Vorbehandeltes Saat- und Pflanzengut kann später in Landwirt, Gartenbau und Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Öle,

Zucker, Stärke, Eiweiss usw., produzieren und zu diesem Zweck angebaut werden. Zu diesen Pflanzen gehören beispielsweise sämtliche Getreidearten, wie Weizen, Roggen, Gerste und Hafer, daneben vor allem Reis, Sorghum, Mais, Baumwolle, Zuckerrüben, Zuckerrohr, Soja, Bohnen, und Erbsen.

Das Gegenmittel kann überall dort eingesetzt werden, wo eine Kulturpflanze der vorgenannten Art vor der schädlichen Wirkung einer Agrarchemikalien geschützt werden soll. Dabei kommen als Agrarchemikalien, wie bereits ausgeführt, in erster Linie Herbizide der verschiedensten Stoffklassen, insbesondere jedoch Halogenacetanilide und Thiolcarbamate in Betracht.

Halogenacetanilide, deren schädigende Wirkung gegenüber Kulturpflanzen mit Hilfe der neuen Oximäther der Formel I aufgehoben werden kann, sind bereits in grosser Zahl bekannt geworden (vgl. z.B. DE 2 305 495, 2 328 340, 2 212 268, 2 726 253 und 2 805 757, sowie US Patentschriften 3 946 044, 4 022 608 und 4 039 314). Solche Halogenacetanilide können durch die folgende allgemeine Formel VII beschrieben werden:

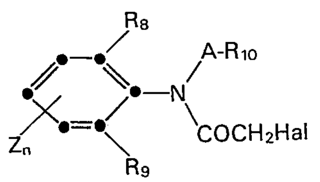

(VIII)

In dieser Formel bedeuten Hal Halogen, insbesondere Chlor oder Brom, $R_8$ und $R_9$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die vorgenannten Reste Z vorzugsweise in 3-Stellung in bezug auf das Stickstoffatom stehen, n 0 bis 3, A Alkylen, insbesondere Methylen, 1,1- und 1,2-Äthylen, wobei 1,2-Äthylen durch 1 - 2 niedere Alkylgruppen substituiert sein kann, und $R_{10}$ niederes Alkoxy, Hydroxycarbonyl, Alkoxycarbonyl, Carbamoyl, N-Alkylcarbamoyl, N,N-Dialkylcarbamoyl, Cyano, ein gegebenenfalls substituierter stickstoffhaltiger heterocyclischer Rest, Alkanoyl, gegebenenfalls substituiertes Benzoyl, gegebenenfalls substituiertes 1,3,4-Oxadiazol-2-yl, 1,3,4-Thiadiazol-2-yl, 1,3,4-Triazol-3-yl oder 1,3,4-Triazol-1-yl bedeutet.

Als einzelne Vertreter solcher Halogenacetanilide seien beispielsweise die folgenden genannt:

N-Äthoxymethyl-N-chloracetyl-2-äthyl-6-methylanilin
N-Chloracetyl-N-methoxymethyl-2,6-diäthylanilin
N-Chloracetyl-N-(2-methoxyäthyl)-2,6-dimethylanilin
N-(2-Allyloxyäthyl)-N-chloracetyl-2,6-dimethylanilin
N-Chloracetyl-N-(2-n-propoxyäthyl)-2,6-dimethylanilin
N-Chloracetyl-N-(2-isopropoxyäthyl)-2,6-dimethylanilin
N-Chloracetyl-N-(2-methoxyäthyl)-2-äthyl-6-methylanilin
N-Chloracetyl-N-(methoxyäthyl)-2,6-diäthylanilin
N-(2-Äthoxyäthyl)-N-chloracetyl-2-äthyl-6-methylanilin
N-Chloracetyl-N-(2-methoxy-1-methyläthyl)-2-methylanilin
N-Chloracetyl-N-(2-methoxy-1-methyläthyl)-2,6-dimethylanilin
N-Chloracetyl-N-(2-methoxy-1-methyläthyl)-2,6-diäthylanilin
N-Chloracetyl-N-(2-methoxy-1-methyläthyl)-2-äthyl-6-methylanilin
N-(2-Äthoxyäthyl)-N-chloracetyl-2,6-diäthylanilin
N-Chloracetyl-N-(2-n-propoxyäthyl)-2-äthyl-6-methylanilin
N-Chloracetyl-N-(2-n-propoxyäthyl)-2,6-diäthylanilin
N-Chloracetyl-N-(2-isopropoxyäthyl)-2-äthyl-6-methylanilin
N-Äthoxycarbonylmethyl-N-chloracetyl-2,6-dimethylanilin
N-Äthoxycarbonylmethyl-N-chloracetyl-2,6-diäthylanilin
N-Chloracetyl-N-methoxycarbonylmethyl-2,6-dimethylanilin
N-Chloracetyl-N-(2,2-diäthoxyäthyl)-2,6-dimethylanilin
N-Chloracetyl-N-(2-methoxy-1-methyläthyl)-2,3-dimethylanilin
N-(2-Äthoxyäthyl)-N-chloracetyl-2-methylanilin
N-Chloracetyl-N-(2-methoxyäthyl)-2-methylanilin
N-Chloracetyl-N-(2-methoxy-2-methyläthyl)-2,6-dimethylanilin
N-(2-Äthoxy-2-methyläthyl)-N-chloracetyl-2-äthyl-6-methylanilin
N-Chloracetyl-N-(1-äthyl-2-methoxyäthyl)-2,6-dimethylanilin
N-Chloracetyl-N-(2-methoxyäthyl)-2-methoxy-6-methylanilin
N-n-Butoxymethyl-N-chloracetyl-2-tert.-butylanilin
N-(2-Äthoxyäthyl-1-methyläthyl)-2,6-dimethylanilin
N-Chloracetyl-N-(2-methoxyäthyl)-2-chlor-6-methylanilin
N-(2-Äthoxyäthyl)-N-chloracetyl-2-chlor-6-methylanilin
N-(2-Äthoxyäthyl)-N-chloracetyl-2,3,6-trimethylanilin
N-Chloracetyl-1-(2-methoxyäthyl)-2,3,6-trimethylanilin
N-Chloracetyl-N-cyanomethyl-2,6-dimethylanilin
N-But-3-in-1-yl-N-chloracetylanilin
N-Chloracetyl-N-propargyl-2-äthyl-6-methylanilin
N-Chloracetyl-N-(1,3-dioxolan-2-ylmethyl)-2,6-dimethylanilin
N-Chloracetyl-N-(1,3-dioxolan-2-ylmethyl)-2-äthyl-6-methylanilin
N-Chloracetyl-N-(1,3-dioxan-2-ylmethyl)-2-äthyl-6-methylanilin
N-Chloracetyl-N-(2-furanylmethyl)-2,6-dimethylanilin
N-Chloracetyl-N-(2-furanylmethyl)-2-äthyl-6-methylanilin
N-Chloracetyl-N-(2-tetrahydrofuranylmethyl)2,6-dimethylanilin
N-Chloracetyl-N-(N-propargylcarbamoylmethyl)-2,6-dimethylanilin

N-Chloracetyl-N-(N,N-dimethylcarbamoylmethyl)--2,6-dimethylanilin
N-(n-Butoxymethyl)-N-chloracetyl-2,6-diäthylanilin
N-(2-n-Butoxyäthyl)-N-chloracetyl-2,6-diäthylanilin
N-Chloracetyl-N-(2-methoxy-1,2-dimethyläthyl)-2,6--dimethylanilin
N-Chloracetyl-N-isopropyl-2,3-dimethylanilin
N-Chloracetyl-N-isopropyl-2-chloranilin
N-Chloracetyl-N-(1H-pyrazol-1-ylmethyl)-2,6-dimethylanilin
N-Chloracetyl-N-(1H-pyrazol-1-ylmethyl)-2-äthyl-6--methylanilin
N-Chloracetyl-N-(1H-1,2,4-triazol-1-ylmethyl)-2,6--dimethylanilin
N-Chloracetyl-N-(1H-1,2,4-triazol-1-ylmethyl)-2,6--diäthylanilin
N-Benzoylmethyl-N-chloracetyl-2,6-dimethylanilin
N-Benzoylmethyl-N-chloracetyl-2-äthyl-6-methylanilin
N-Chloracetyl-N-(5-methyl-1,3,4-oxadiazol-2-yl)--2,6-diäthylanilin
N-Chloracetyl-N-(5-methyl-1,3,4-oxadiazol-2-yl)-2--äthyl-6-methylanilin
N-Chloracetyl-N-(5-methyl-1,3,4-oxadiazol-2-yl)-2--tert.-butylanilin
N-Chloracetyl-N-(4-chlorbenzoylmethyl)-2,6-dimethylanilin
N-Chloracetyl-N-(1-methyl-5-methylthio-1,3,4-triazol-2-ylmethyl)-2,6-diäthylanilin.

Weitere Halogenacetanilide, deren schädigende Wirkung auf Kulturpflanzen durch die neuen Oximäther der Formel I aufgehoben werden kann, sind in R. Wegler, Chemie der Pflanzenschutz- und Schädlingsbekämpfungsmittel, Bd. 8, Seitenb 90-83 und Seiten 322-327 aufgeführt.

Herbizid wirksame Thiolcarbamate, vor deren phytotoxischen Wirkung Kulturpflanzen durch die neuen Oximäther der Formel I geschützt werden können, sind ebenfalls bereits in grosser Zahl bekannt geworden (vgl. z.B. US-Patentschriften 2 913 853, 3 037 853, 3 175 897, 3 185 720, 3 198 786, 3 582 314 und 3 846 115). Die Schutzwirkung der neuen Oximäther der Formel I lässt sich insbesondere beim Einsatz von Thiolcarbamaten in Getreide, Reis oder veredelter Sorghum-Hirse vorteilhaft ausnützen.

Die Thiolcarbamate, vor deren phytotoxischer Wirkung Kulturpflanzen, wie Getreide, Reis und veredelte Sorghum-Hirse bevorzugt geschützt werden können, entsprechen den allgemeinen Formeln IX und X:

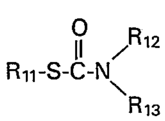 (IX) 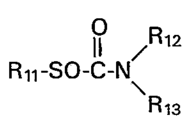 (X)

In diesen Formeln bedeutet $R_{11}$ niederes Alkyl, Alkenyl, Chlorallyl, Dichlorallyl, Trichlorallyl, Benzyl oder 4-Chlorbenzyl, $R_{12}$ $C_2$-$C_4$- Alkyl und $R_{13}$ $C_2$-$C_4$-Alkyl oder Cyclohexyl, wobei die Reste $R_{12}$ und $R_{13}$ zusammen mit dem Stickstoffatom, an das sie gebunden sind, einen Hexahydro-1H-azepin-, Dekahydrochinolin- oder 2-Methyldekahydrochinolin-Ring bilden können.

Als einzelne Vertreter solcher Thiolcarbamate seien beispielsweise die folgenden genannt:
S-Äthyl-N,N-dipropylthiocarbamat,
S-Äthyl-N,N-diisobutylthiocarbamat,
S-2,3-Dichlorallyl-N,N-diisopropylthiolcarbamat,
S-Propyl-N-butyl-N-äthylthiolcarbamat,
S-2,3,3-Trichlorallyl-N,N-diisopropylthiocarbamat,
S-Propyl-N,N-dipropylthiolcarbamat,
S-Äthyl-N-äthyl-N-cyclohexylthiolcarbamat,
S-Äthyl-N-hexahydro-1H-azepin-1-carbothioat,
S-Isopropyl-N,N-hexamethylen-thiolcarbamat,
S-(p-Chlorbenzyl)-N,N-diäthylthiolcarbamat,
N-Äthylthiocarbonyl-cis-decahydrochinolin,
N-Propylthiocarbonyl-decahydrochinaldin,
S-Äthyl-N,N-bis(n-butyl)-thiolcarbamat,
S-tert.-Butyl-N,N-bis(n-propyl)-thiocarbamat.

Daneben kommen Herbizide anderer Stoffklassen, wie beispielssweise die folgenden Substanzen in Betracht:

*Triazine und Triazinone:* 2,4-Bis(isopropylamino)--6-methylthio-1,3,5-triazin («Prometryn»), 2,4-bis-(äthylamino)-6-methylthio-1,3,5-triazin («Simetryn»), 2-(1',2'-Dimethylpropylamino)-4-äthylamino--6-methylthio-1,3,5-triazin («Dimethametryn»), 4--Amino-6-tert.-butyl-4,5-dihydro-3-methylthio-1,2,4--triazin-5-on («Metribuzin»).

*Phenylharnstoffe:* N-(3',4'-Dimethylbenzyl)-N'-4--tolyl-harnstoff (® Dimuron), N-(3'-Chlor-4'-isopropylphenyl)-N',N'-(3-methyl-pentamethylen-1,5-yl)--harnstoff.

*Chloracetamide:* N-[1-Isopropyl-2-methylpropen-1--yl-(1)]-N(2'-methoxyäthyl)-chloracetamid.

*Diphenyläther und Nitrodiphenyläther:* 2,4-Dichlorphenyl-4'-nitrophenyläther («Nitrofen»), 2--Chlor-1-(3'-äthoxy-4'-nitrophenoxy)-4-trifluormethyl-benzol («Oxyfluorfen»), 2',4,-Dichlorphenyl-3--methoxy-4-nitrophenyläther («Chlormethoxynil»), 2-[4'-(2'',4''-Dichlorphenoxy)propionsäure-methylester, N-(2'-Phenoxyäthyl)-2-[5'-(2''-chlor-4''-trifluormethylphenoxy)-phenoxy]-propionsäureamid.

*Benzoesäurederivate:* Methyl-5-(2',4'-dichlorphenoxy)-2-nitrobenzoat («Bifenox»), 5-(2'-Chlor-4'-trifluormethylphenoxy)-2-nitrobenzoesäure («Acifluorfen»), 2,6-Dichlorbenzonitril («Dichlorbenil»).

*Nitroaniline:* 2,6-Dinitro-N,N-dipropyl-4-trifluormethylanilin («Trifluralin»). N-(1'-Äthoxypropyl)-2,6--dinitro-3,4-xylidin («Pendimethalin»).

*Oxadiazolone:* 5-tert.-Butyl-3-(2',4'-dichlor-5'-isopropoxyphenyl)-1,3,4-oxadiazol-2-on («Oxadiazon»).

*Phophate:* S-2-Methylpiperidino-carbonylmethyl--O,O-dipropyl-phosphorodithioat («Piperophos»).

*Pyrazole:* 1,3-Dimethyl-4-(2',4'-dichlorbenzoyl)-5--4'-tolylsulfonyloxy)-pyrazol.

Ferner die Derivate der α-[4-(Phenoxy)phenoxy]--propionsäure oder der α-[4-(Pyridyl-2-oxy)phenoxy]-propionsäure welche der Formel XI entsprechen.

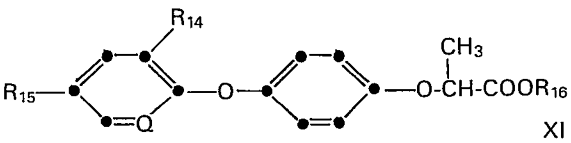

XI

worin

$R_{14}$ Wasserstoff oder Halogen,

$R_{15}$ Wasserstoff, Halogen oder Trifluormethyl,

Q das Fragment =N- oder =CH-,

$R_{16}$ $C_1$-$C_4$-Alkyl. welches unsubstituiert oder durch $C_1$-$C_4$-Alkoxy substituiert ist, $C_3$-$C_4$-Alkenyl, $C_3$-$C_4$-Alkinyl oder $-N=C\langle{}^{R_{17}}_{R_{18}}$

$R_{17}$ $C_1$-$C_4$-Alkyl, $R_{14}$ $C_1$-$C_4$-Alkyl oder $R_{17}$ und $R_{18}$ gemeinsam $C_1$-$C_5$-Alkylen bedeuten.

Die angewendete Menge der Gegenmittel schwankt zwischen etwa 0,01 und etwa 15 Gewichtsteilen pro Gewichtsteil Herbizid. Man ermittelt von Fall zu Fall, d.h. je nach verwendeten Herbizid-Typ, welches Verhältnis in bezug auf optimale Wirkung bei der speziellen Kulturpflanze das geeignetste ist.

Die Oximäther der Formel I eignen sich ausserdem zur Beeinflussung des Pflanzenwachstums. Sie stimulieren beispielsweise das Wurzelwachstum von Keimlingen, insbesondere Getreide-Mais und Reissamen, so dass solche Kulturen forciert angebaut werden können, z.B. im Falle ungünstiger klimatischer Bedingungen oder intensiver Fruchtfolge.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Di-octylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonid oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind z.B. die Alkali-, Erdalkali- oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Äthylenoxyd-Adduktes in Frage.

Als nichtionisches Tensid kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Äthylenglykoläthergruppen und 10 bis 1000 Propylenglykoläthergruppen enthaltenden Polyäthylenoxidaddukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglycoläther, Polypropylen-Polyäthylenoxyaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor. z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

«Mc Cutcheon's Detergens end Emulsifiers Annual» Mc. Publishing Corp., Ringwood, New Jersey, 1979 Sisely and Wood, «Encyclopedia of Surface Active Agents», Chemical Publishing Co., Inc. New York, 1964.

Diese Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Centigraden angegeben, die Druckangaben sind in Millibar und die Teile in Prozentangaben beziehen sich auf das Gewicht.

*Beispiel 1*

*Herstellung von N-(1,3-Dioxolan-5-yl-methoxy)-imino-para-chlorphenylacetonitril (Verbindung No. 4)*

In einem 100 ml Rundkolben werden 22,6 g (0,10 Mol) 2-(4-Chlorphenyl)-2-hydroximino-acetonitril Natriumsalz in 50 ml Dimethylsulfoxid gelöst. Dazu tropft man unter Rühren 13,4 g (0,11 Mol) 5-Chlormethyl-1,3-dioxolan und rührt 4 Stunden bei 60 bis 70°C Innentemperatur nach. Dann wird die entstandene Suspension abgekühlt und auf eine Eis-Wasser-Mischung gegossen. Aus der erhaltenen Mischung wird das Reaktionsprodukt durch Extraktion mit Methylenchlorid gewonnen. Der Extrakt wird über Natriumsulfat getrocknet und das Lösungsmittel abgedampft. Als Rohprodukt wird ein orangefarbenes Öl erhalten. Das Reaktionsprodukt wird im Hochvakuum bei 130°C Badtemperatur andestilliert. Es werden so 15,8 g (59,2% der Theorie) N-(1,3-Dioxolan-5-yl-methoxy)-imino-para-chlorphenyl-acetonitril als farbloses Öl vom Brechungsindex $n_D^{20}$ 1.5690 erhalten.

*Beispiel 2*

*Herstellung von N-(2-Methyl-1,3-dioxolan-5-yl)-methoxy-imino-para-chlorphenylacetonitril (Verbindung Nr. 5)*

In einem 100 ml Rundkolben löst man 22,6 g (0,10 Mol) 2-(4-Chlorphenyl)-2-hydroximino-acetonitril Natriumsalz in 50 ml Dimethylsulfoxyd. Dazu tropft man unter Rühren 14,9 g (0,11 Mol) 5-Chlormethyl-2-methyl-1,3-dioxolan und rührt 4 Stunden bei 60 bis 70°C Innentemperatur nach. Dann wird die entstandene Suspension abgekühlt und auf eine Eis-Wasser-Mischung gegossen. Aus der erhaltenen Mischung wird das Reaktionsprodukt durch Extraktion mit Methylenchlorid gewonnen. Der Extrakt wird über Natriumsulfat getrocknet und das Lösungsmittel abgedampft. Als Rohprodukt wird ein organgefarbenes Öl erhalten. Das Rohprodukt wird im Hochvakuum bei 130°C Badtemperatur andestilliert. Es werden so 17 g (60,5% der Theorie) N-(2-Methyl-1,3-dioxolan-5-yl)-methoxy-imino-para-chlorphenylacetonitril als farbloses Öl vom Brechungsindex $n_D^{20}$ 1.5880 erhalten.

In analoger Weise zu diesen Beispielen werden folgende Verbindungen hergestellt:

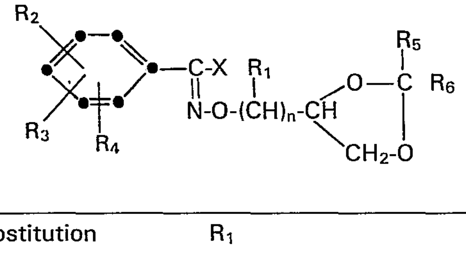

| No. | X | Phenylsubstitution (R2 R3 R4) | R1 (CH)n | CR5R6 | phys. Daten |
|---|---|---|---|---|---|
| 1 | $CF_3$ | — | $CH_2$ | $CH_2$ | Sdp. 82-84°/ 0,08 mbar |
| 2 | $CF_3$ | 4-Cl | $CH_2$ | $CH_2$ | $n_D^{20}$ 1.4985 |

| No. | X | Phenylsubstitution ($R_2$ $R_3$ $R_4$) | $R_1$ $(CH)_n$ | $CR_5R_6$ | phys. Daten |
|---|---|---|---|---|---|
| 3 | CN | — | $CH_2$ | $CH_2$ | $n_D^{20}$ 1.5500 |
| 4 | CN | 4-Cl | $CH_2$ | $CH_2$ | $n_D^{20}$ 1.5690 |
| 5 | CN | 4-Cl | $CH_2$ | $CHCH_3$ | $n_D^{20}$ 1.5580 |
| 6 | CN | — | $CH_2$ | $CHCH_3$ | $n_D^{20}$ 1.5370 |
| 7 | CN | — | $CH_2$ | $CHC_2H_5$ | $n_D^{20}$ 1.5330 |
| 8 | CN | — | $CH_2$ | $C(CH_3)_2$ | Smp. 62-63° |
| 9 | CN | — | $CH_2$ | $CH(C_3H_7i)$ | $n_D^{20}$ 1.5280 |
| 10 | CN | 2-Cl | $CH_2$ | $CH_2$ | $n_D^{20}$ 1.5660 |
| 11 | CN | 3-Cl | $CH_2$ | $CH_2$ | $n_D^{20}$ 1.5650 |
| 12 | CN | 2-F | $CH_2$ | $CH_2$ | $n_D^{20}$ 1.5400 |
| 13 | CN | 2-F | $CH_2$ | $CH(CH_3)$ | $n_D^{20}$ 1.5290 |
| 14 | CN | 4-F | $CH_2$ | $CH_2$ | $n_D^{20}$ 1.5530 |
| 15 | CN | 2,4-$Cl_2$ | $CH_2$ | $CH_2$ | $n_D^{20}$ 1.5770 |
| 16 | CN | 4-Cl | $CH_2$ | $CHC_2H_5$ | $n_D^{20}$ 1.5500 |
| 17 | CN | 2-$CH_3$ | $CH_2$ | $CH_2$ | |
| 18 | CN | 3-$CH_3$ | $CH_2$ | $CH_2$ | $n_D^{20}$ 1.5490 |
| 19 | CN | 4-$CH_3$ | $CH_2$ | $CH_2$ | $n_D^{20}$ 1.5490 |
| 20 | CN | 2-$OCH_3$ | $CH_2$ | $CH_2$ | $n_D^{20}$ 1.5350 |
| 21 | CN | 3-$OCH_3$ | $CH_2$ | $CH_2$ | $n_D^{20}$ 1.5540 |
| 22 | CN | 4-$CH_3O$ | $CH_2$ | $CH_2$ | $n_D^{20}$ 1.5670 |
| 23 | CN | 4-Br | $CH_2$ | $CH_2$ | $n_D^{20}$ 1.5820 |
| 24 | CN | 3,4 $Cl_2$ | $CH_2$ | $CH_2$ | wachsartig |
| 25 | CN | 2,3-CH=CH-CH=CH- | $CH_2$ | $CH_2$ | Öl |
| 26 | $CF_3$ | 2-Cl | $CHCH_3$ | $C(CH_3)_2$ | |
| 27 | CN | — | $CHCH_3$ | $CH_2$ | |
| 28 | $CF_3$ | — | — | $C(CH_3)_2$ | |
| 29 | CN | 3-Cl | — | $CH_2$ | |
| 30 | CN | 4-Cl | — | $CHC_2H_5$ | |
| 31 | CN | 4-Cl | — | $C(C_2H_5)_2$ | |
| 32 | CN | 4-$CF_2$ | — | $CH_2$ | |
| 33 | $CF_3$ | 4-Cl | — | $C(CH_3)_2$ | |
| 34 | $CF_3$ | 4-Cl | — | $C(C_2H_5)_2$ | |
| 35 | $CF_3$ | 4-Cl | — | $C(CH_3)C_2H_5$ | |
| 36 | $CF_3$ | 4-$CH_3$ | — | $CH_2$ | |
| 37 | $CF_3$ | 4-$CH_3$ | — | $C(CH_3)_2$ | |
| 38 | $CF_3$ | 4-$CH_3$ | $CH(CH_3)$ | $CH(CH_3)$ | |
| 39 | $CF_3$ | 4-$CH_3$ | $CH(CH_3)$ | $C(CH_3)_2$ | |
| 40 | CN | 2-F | — | $CH_2$ | |
| 41 | CN | 2-Cl | $CH_2$ | $CH(CH_3)$ | |
| 42 | CN | 2-Cl | $CH_2$ | $CHC_2H_5$ | |
| 43 | CN | 2-Cl | $CH_2$ | $C(CH_3)_2$ | |
| 44 | CN | 2-Cl | $CH_2$ | $CH\ C_3H_7i$ | |
| 45 | CN | 3-Cl | $CH_2$ | $CHCH_3$ | |
| 46 | CN | 3-Cl | $CH_2$ | $CH\ C_2H_5$ | |
| 47 | CN | 3-Cl | $CH_2$ | $C(CH_3)_2$ | |
| 48 | CN | 3-Cl | $CH_2$ | $CHC_3H_7i$ | |
| 49 | CN | 4-Cl | $CH_2$ | $C(CH_3)_2$ | |

| No. | X | Phenylsubstitution ($R_2$ $R_3$ $R_4$) | $R_1$ $(CH)_n$ | $CR_5R_6$ | phys. Daten |
|---|---|---|---|---|---|
| 50 | CN | 4-Cl | $CH_2$ | $CHC_3H_7i$ | |
| 51 | CN | 2-F | $CH_2$ | $CHC_2H_5$ | |
| 52 | CN | 2-F | $CH_2$ | $C(CH_3)_2$ | |
| 53 | CN | 2-F | $CH_2$ | $CHC_3H_7i$ | |
| 54 | CN | 4-F | $CH_2$ | $CHCH_3$ | |
| 55 | CN | 4-F | $CH_2$ | $CHC_2H_5$ | |
| 56 | CN | 4-F | $CH_2$ | $C(CH_3)_2$ | |
| 57 | CN | 4-F | $CH_2$ | $CHC_3H_7i$ | |
| 58 | CN | 2,4-Cl | $CH_2$ | $CHCH_3$ | |
| 59 | CN | 2,4-$Cl_2$ | $CH_2$ | $CHC_2H_5$ | |
| 60 | CN | 2,4-$Cl_2$ | $CH_2$ | $C(CH_3)_2$ | |
| 61 | CN | 2,4-$Cl_2$ | $CH_2$ | $CHC_3H_7i$ | |
| 62 | CN | 2-$CH_3$ | $CH_2$ | $CHCH_3$ | |
| 63 | CN | 2-$CH_2$ | $CH_2$ | $CHC_2H_5$ | |
| 64 | CN | 2-$CH_3$ | $CH_2$ | $CHC_3H_7i$ | |
| 65 | CN | 2-$CH_3$ | $CH_2$ | $C(CH_3)_2$ | |
| 66 | CN | 4-Br | $CH_2$ | $CHCH_3$ | |
| 67 | CN | 4-Br | $CH_3$ | $C(CH_3)_2$ | |
| 68 | CN | 4-Br | $CH_2$ | $CHC_2H_5$ | |
| 69 | CN | 4-$OCH_3$ | $CH_2$ | $C(CH_3)_2$ | |
| 70 | CN | 4-$OCH_3$ | $CH_2$ | $COC_2H_5$ | |
| 71 | CN | 3-$OCH_3$ | $CH_2$ | $C(CH_3)_2$ | |
| 72 | CN | 4-$CH_3$ | $CH_2$ | $C(CH_3)_2$ | |
| 73 | $CF_3$ | — | $CH_2$ | $C(CH_3)_2$ | |
| 74 | $CF_3$ | — | $CH_2$ | CH-$C_6H_5$ | |
| 75 | $CF_3$ | 3,4 $Cl_2$ | $CH(CH_3)$ | $CHC_6H_4Cl(4)$ | |
| 76 | $CF_3$ | 4-Cl | $CH_2$ | $CH_2$ | |
| 77 | $CF_3$ | 4-Cl | $CH_2$ | $CHCH_3$ | |
| 78 | CN | $2CH_3$ 4,6 $Cl_2$ | $CH_2$ | $CHC_2H_5$ | |
| 79 | CN | $2CH_3$ 4,6 $Cl_2$ | $CH(CH_3)$ | $CH_2$ | |
| 80 | $CF_3$ | $2CH_3$ 4,5 $Cl_2$ | $CH_2$ | $C(CH_3)_2$ | |
| 81 | CN | — | $CH_2$ | $CHCF_3$ | |
| 82 | CN | 4-Cl | $CH_2$ | $CHCH_2Cl$ | |
| 83 | CN | — | $CH(CH)_3CH(CH_3)$ | $CH_2$ | |
| 84 | CN | 4-Cl | $CH(CH_3)CH(CH_3)$ | $C(CH_3)_2$ | |
| 85 | $CF_3$ | 4-Cl | $C_2H_4$ | $CH_2$ | |
| 86 | CN | 4-Cl | $C_2H_4$ | $CH_2$ | |
| 87 | CN | — | $CH_2$ | $CH(CCl_3)$ | |
| 88 | CN | 4-Cl | $CH_2$ | $CH(CCl_3)$ | |
| 89 | CN | 4-F | $CH_2$ | $CH(CCl_3)$ | |
| 90 | CN | 4-$CH_3$ | $CH_2$ | CH-$CH_3$ | $n_D^{24}$ 1.5400 |
| 91 | CN | 4-$CH_3$ | $CH_2$ | CH-$C_2H_5$ | $n_D^{24}$ 1.5370 |
| 92 | CN | 4-$CH_3$ | $CH_2$ | $C(CH_3)_2$ | $n_D^{24}$ 1.5340 |
| 93 | CN | 4-$CH_3$ | $CH_3$ | CH-$CH(CH_3)_2$ | $n_D^{24}$ 1.5315 |
| 94 | CN | 4-Cl | $CH_2$ | $C(CH_3)_2$ | $n_D^{24}$ 1.5485 |
| 95 | CN | — | $CH_2$ | Cyclohexyl | |
| 96 | CN | — | $CH_2$ | Cyclopentyl | |

*Beispiel 3*

*Formulierungsbeispiele für Wirkstoffe der Formel I oder Mischungen dieser Wirkstoffe mit Herbiziden*

a) *Spritzpulver*

| | a) | b) | c) |
|---|---|---|---|
| Wirkstoff der Formel I oder Mischung mit Herbizid | 20% | 60% | 0,5% |
| Na-Ligninsulfonat | 5% | 5% | 5% |
| Na-Laurylsulfat | 3% | — | — |
| Na-Diisobutylnaphthalinsulfonat | — | 6% | 6% |
| Octylphenolpolyäthylenglykoläther (7-8 Mol) AeO) | — | 2% | 2% |
| Hochdisperse Kieselsäure | 5% | 27% | 27% |
| Kaolin | 67% | — | — |
| Natriumchlorid | — | — | 59,5% |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) *Emulsion-Konzentrat*

| | a) | b) |
|---|---|---|
| Wirkstoff der Formel I oder Mischung mit Herbizid | 10% | 1% |
| Octylphenolpolyäthylenglykoläther (4-5 Mol AeO) | 3% | 3% |
| Ca-Dodecylbenzolsulfonat | 3% | 3% |
| Ricinusölpolyglykoläther (36 Mol AeO) | 4% | 4% |
| Cyclohexanon | 30% | 10% |
| Xylolgemisch | 50% | 79% |

Aus diesem Konzentrat können durch Verdünnen mit Wasser Emulsionen jeder gewünschten Konzentration hergestellt werden.

c) *Stäubemittel*

| | a) | b) |
|---|---|---|
| Wirkstoff der Formel I oder Mischung mit Herbizid | 0,1% | 1% |
| Talkum | 99,9% | — |
| Kaolin | — | 99% |

Man erhält anwendungsfertige Stäubemittel, indem der Wirkstoff mit dem Träger vermischt und auf einer geeigneten Mühle vermahlen wird.

d) *Extruder-Granulat*

| | a) | b) |
|---|---|---|
| Wirkstoff der Formel I oder Mischung mit Herbizid | 10% | 1% |
| Na-Ligninsulfonat | 2% | 2% |
| Carboxymethylcellulose | 1% | 1% |
| Kaolin | 87% | 96% |

Der Wirkstoff wird mit den Zusatzstoffen vermischt, vermahlen und mit Wasser angefeuchtet. Dieses Gemisch wird extrudiert und anschliessend im Luftstrom getrocknet.

e) *Umhüllungs-Granulat*

| | |
|---|---|
| Wirkstoff der Formel I oder Mischung mit Herbizid | 3% |
| Polyäthylenglykol (MG 200) | 3% |
| Kaolin | 94% |

Der fein gemahlene Wirkstoff wird in einem Mischer auf das mit Polyäthylenglykol angefeuchtete Kaolin gleichmässige aufgetragen. Auf diese Weise erhält man staubfreie Umhüllungs-Granulate.

f) *Suspensions-Konzentrat*

| | a) | b) |
|---|---|---|
| Wirkstoff der Formel I oder Mischung mit Herbizid | 40% | 5% |
| Äthylenglykol | 10% | 10% |
| Nonylphenolpolyäthylenglykoläther (15 Mol AeO) | 6% | 1% |
| Na-Ligninsulfonat | 10% | 5% |
| Carboxymethylcellulose | 1% | 1% |
| 37%ige wässrige Formaldehyd-Lösung | 0,2% | 0,2% |
| Silikonöl in Form einer 75%igen wässrigen Emulsion | 0,8% | 0,8% |
| Wasser | 32% | 77% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) *Salzlösung*

| | |
|---|---|
| Wirkstoff der Formel I oder Mischung mit Herbizid | 5% |
| Isopropylamin | 1% |
| Octylphenolpolyäthylenglykoläther (78 Mol AeO) | 3% |
| Wasser | 91% |

*Biologische Beispiele*

Die Fähigkeit der Verbindungen der Formel I, Kulturpflanzen vor der phytotoxischen Wirkung starker Herbizide zu schützen, kann aus den folgenden Beispielen ersehen werden. In den Versuchsbeschreibungen werden die Verbindungen der Formel I als Antidote (Gegenmittel) bezeichnet.

*Beispiel 4*

*Versuch mit Antidote und Herbizid in Sorghum. Applikation von Herbizid und Antidote als Tankmischung im Vorauflauf.*

Plastikcontainer (25 cm lang × 17 cm breit × 12 cm hoch) werden mit sandiger Lehmerde gefüllt und Sorghumsamen der Sorte Funk G 522 eingesät. Nach dem Bedecken der Samen wird die als Safener zu prüfende Substanz zusammen mit dem Herbizid in verdünnte Lösung als Tankmischung auf die Bodenoberfläche gesprüht. 30 Tage nach der Applikation wird die Schutzwirkung des Safeners in Prozent bonitiert. Als Referenz dienen dabei die mit dem Herbizid allein behandelten Pflanzen sowie die vollständig unbehandelte Kontrolle.

*Herbizid:* N-Chloracetyl-N-(2-methoxy-1-methyläthyl)-2-äthyl-6-methylanilin («Metolachlor»)

*Gegenmittel:* N-(4-Methyl-1,3-dioxolan-5-yl-methoxy)-imino-para-chlor-phenylacetonitril (Verbindung Nr. 5)

| | Herbizid | Gegenmittel Verb. Nr. 5 | relative Schutzwirkung (Verbesserung der Verträglichkeit) |
|---|---|---|---|
| Aufwand-menge | 1 | 1 | 50% |
| kg/ha | 1 | 0,5 | 37,5% |

*Beispiel 5*

*Versuch mit Antidote und Herbizid in Sorghum im Vorauflaufverfahren. Applikation der Antidote und des Herbizides als Tank-Mix.*

Töpfe (oberer Durchmesser 6 cm) werden mit sandiger Lehmerde gefüllt und Sorghumsamen der Sorte G 522 werden eingesät. Nach dem Bedecken der Samen wird die als Safener zu prüfende Substanz zusammen mit dem Herbizid in verdünnter Lösung als Tankmischung auf die Bodenoberfläche versprüht. 21 Tage nach der Herbizidapplikation wird die Schutzwirkung des Safeners in Prozent bonitiert. Als Referenzen dienen dabei die mit Herbizid allein behandelten Pflanzen sowie die vollständig unbehandelte Kontrolle.

Die Ergebnisse sind wie folgt:

*Herbizid:* 2-Chlor-6'-äthyl-N-(2" methoxy-1" -methyläthyl)-acet-o-toluidid «Metolachlor»

| Aufwandmenge Antidote | | Herbizid | relative Schutzwirkung |
|---|---|---|---|
| Nr. 3 | 1,5 kg/ha | 1,5 kg/ha | 25% |
| Nr. 4 | 1,5 kg/ha | 1,5 kg/ha | 38% |
| Nr. 5 | 1,5 kg/ha | 1,5 kg/ha | 38% |
| Nr. 13 | 1,5 kg/ha | 1,5 kg/ha | 25% |
| Nr. 90 | 1,5 kg/ha | 1,5 kg/ha | 25% |
| Nr. 92 | 1,5 kg/ha | 1,5 kg/ha | 38% |

*Beispiel 6*

*Versuch mit Antidote und Herbizid in Weizen im Nachauflauf. Applikation von Antidote und Herbizid als Tank-mix.*

Weizensamen der Sorte «Farnese» werden in Plastiktöpfe (oberer Durchmesser 11 cm), die 0,5 ℓ Erde enthalten, im Gwächshaus ausgesät. Nach dem Bedecken der Samen wird die als Safener zu prüfende Substanz zusammen mit dem Herbizid als Tankmischung im Nachauflauf appliziert. 20 Tage nach der Applikation wird die Schutzwirkung des Safeners in Prozent bonitiert. Als Referenzen dienen dabei die mit Herbizid allein behandelten Pflanzen sowie die vollständig unbehandelte Kontrolle.

Die Ergebnisse sind wie folgt:

*Herbizid:* α-[4-(2',4' -Dichlorpyridyl-2' -oxy)phenoxy]-propionsäure-propinylester

| Aufwandmenge Antidote | | Herbizid | relative Schutzwirkung |
|---|---|---|---|
| Nr. 8 | 1,5 kg/ha | 0,75 kg/ha | 25% |
| Nr. 9 | 1,5 kg/ha | 0,75 kg/ha | 25% |
| Nr. 25 | 1,5 kg/ha | 0,75 kg/ha | 25% |

*Beispiel 7*

*Versuch mit Antidote und Herbizid in Reis Applikation des Antidote durch Samenquellung. Applikation des Herbizides im Vorauflauf auf feuchten Boden.*

Reissamen werden während 48 Stunden mit Lösungen der als Safener zu prüfenden Substanz von 100 ppm getränkt. Anschliessend werden die Samen etwa 2 Stunden trocknen gelassen, bis sie nicht mehr kleben. Plastikcontainer (25 cm lang, 17 cm breit und 12 cm hoch) werden bis 2 cm unter dem Rand mit sandigem Lehm gefüllt. Die vorgequollenen Samen werden auf der Bodenoberfläche des Containers gesät und nur ganz schwach gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstum sukzessiv erhöht. 18 Tage nach der Herbizidapplikation wird die Schutzwirkung des Safeners in Prozent bonitiert. Als Referenz dienen dabei die mit dem Herbizid allein behandelten Pflanzen sowie die vollständig unbehandelte Kontrolle.

Das Ergebnis ist wie folgt:

*Herbizid:* 2-Chlor-2,6' -diäthyl-N-(2" -propyläthyl)--acetanilid, «Pretilachlor»

| Aufwandmenge Antidote | | Herbizid | relative Schutzwirkung |
|---|---|---|---|
| Nr. 11 | 100 ppm | 0,25 kg/ha | 25% |

*Beispiel 8*

*Versuch mit Antidote und Herbizid in Soja im Vorauflauf. Applikation von Antidote und Herbizid als Tankmix.*

Töpfe (oberer Durchmesser 6 cm) werden mit sandiger Lehmerde gefüllt und Sojasamen der Sorte «Hark» werden eingesät. Nach dem Bedecken der Samen wird die als Safener zu prüfende Substanz zusammen mit dem Herbizid in verdünnte Lösung als Tankmischung auf die Bodenoberfläche versprüht. 21 Tage nach der Herbizidapplikation wird die Schutzwirkung des Safeners in Prozent bonitiert. Als Referenzen dienen dabei die mit Herbizid allein behandelten Pflanzen sowie die vollständig unbehandelte Kontrolle.

Das Ergebnis ist wie folgt:

*Herbizid:* 4-Amino-6-tert.-butyl-4,5-dihydro-3-methylthio-1,2,4-triazin-5(4H)-on, «Metribuzin»

| Aufwandmenge Antidote | Herbizid | relative Schutzwirkung |
|---|---|---|
| Nr. 25 1,5 kg/ha | 0,75 kg/ha | 25% |

*Beispiel 9*

*Versuch zur Stimulierung des Wurzelwachstums von keimenden Weizensamen.*

Zur Bestimmung der Förderung des Wurzelwachstums wird im Gewächshaus Weizensamen, der mit 25 mg Prüfsubstanz pro kg Saatgut gebeizt ist, sowie unbehandelter Weizensamen derselben Gattung in flache mit Erde gefüllte Plastikzylinder (5 cm × 30 cm) gesät. Pro Zylinder kommen 10 Samen. Die Zylinder werden dann in Klimakammern unter kontrollierten Bedingungen gehalten. Nach 10 Tage werden die Keimlinge sorgfältig aus der Erde gewaschen. Man bestimmt die Länge der Wurzeln und deren Trockengewicht und vergleicht mit den von den Keimlingen des unbehandelten Weizens erhaltenen Werten. Die Verbindungen Nr. 19, 90, 91 und 92 zeigen gegenüber den Kontrollen bis zu 17% mehr Längenwachstum und bis zu 21% mehr Gewicht.

**Patentansprüche**

1. Oximäther der Formel I

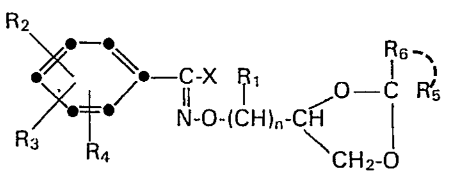

(I)

in welcher n 1 oder 2, $R_1$ und $R_2$ je Wasserstoff oder $C_1$-$C_4$ Alkyl, $R_3$ und $R_4$ je Wasserstoff, Halogen, $C_1$-$C_4$ Alkyl, $C_1$-$C_4$ Halogenalkyl, $C_1$-$C_4$ Alkoxy, $C_1$-$C_4$ Halogenalkoxy, $C_1$-$C_4$ Alkylthio, $C_1$-$C_4$ Halogenalkylthio, $C_1$-$C_4$ Alkylsulfinyl, $C_1$-$C_4$ Alkylsulfonyl, $C_1$-$C_4$ Halogenalkylsulfinyl, $C_1$-$C_4$ Halogenalkylsulfonyl oder Nitro, $R_5$ und $R_6$ einzeln je Wasserstoff, $C_1$-$C_4$ Alkyl, $C_1$-$C_4$ Halogenalkyl, Phenyl, unsubstituiert oder substituiert durch Halogen, $C_1$-$C_4$ Alkyl, $C_1$-$C_4$ Halogenalkyl, $C_1$-$C_4$ Alkoxy, $C_1$-$C_4$ Halogenalkoxy, $C_1$-$C_4$ Alkylthio, $C_1$-$C_4$ Alkylsulfinyl, $C_1$-$C_4$ Alkylsulfonyl, Carboxyl, Carbamoyl, $C_1$-$C_4$-Alkylcarbamoyl, Nitro oder Cyan, $R_5$ und $R_6$ zusammen auch eine 2 bis 6gliedrige Alkylen- oder Alkenylenkette, die durch $C_1$-$C_4$ Alkylreste substituiert sein kann, X Wasserstoff, Cyan, Nitro, Chlor, $C_1$-$C_4$ Alkyl, $C_1$-$C_4$ Halogenalkyl, $C_3$-$C_6$ Cycloalkyl, Carboxyl, Carbamoyl, $C_1$-$C_4$ Alkylcarbonyl, $C_1$-$C_4$ Alkoxycarbonyl, $C_1$-$C_4$ Alkylcarbamoyl bedeuten.

2. Oximäther gemäss Anspruch 1 der Formel Ia

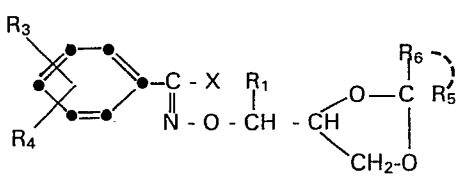

(Ia)

worin n. $R_3$, $R_5$, $R_6$ und X die im Anspruch 1 gegebene Bedeutung haben, $R_4$ Halogen und $R_1$ Wasserstoff oder Methyl bedeuten.

3. Oximäther gemäss Anspruch 1 der Formel Ib

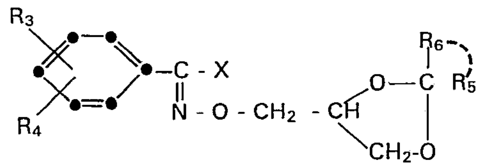

(Ib)

worin $R_3$, $R_5$ und $R_6$ die im Anspruch 1 gegebene Bedeutung haben und $R_4$ Wasserstoff oder Halogen bedeutet.

4. Oximäther gemäss Anspruch 1 der Formel Ic

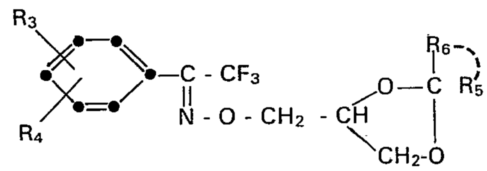

(Ic)

worin $R_3$, $R_5$ und $R_6$ die im Anspruch 1 gegebene Bedeutung haben und $R_4$ Wasserstoff oder Halogen bedeutet.

5. N-(1,3-Dioxolan-5-yl-methoxy)-imino-para--chlorphenylacetonitril gemäss Anspruch 1.

6. N-(2-Methyl-1,3-dioxolan-5-yl-methoxy)-imino-para-chlorphenylacetonitril gemäss Anspruch 1.

7. 1-Phenyl-1-(1,3-dioxolan-5-yl-methoximino)--2,2,2-trifluoräthan gemäss Anspruch 1.

8. 1-(4-Chlorphenyl)-1-(1,3-dioxolan-5-yl-methoximino)-2,2,2-trifluoräthan gemäss Anspruch 1.

9. N-(2,2-Dimethyl-1,3-dioxolan-5-yl-methoxy)--imino-para-chlorphenylacetonitril gemäss Anspruch 1.

10. N-(1,3-Dioxolan-5-yl-methoxy)-imino-phenylacetonitril gemäss Anspruch 1.

11. N-(2-Methyl-1,3-dioxolan-5-yl-methoxy)-imino-ortho-fluorphenylacetonitril gemäss Anspruch 1.

12. N-(2-Methyl-1,3-dioxolan-5-yl-methoxy)-imino-para-tolylacetonitril gemäss Anspruch 1.

13. N-(2,2-Dimethyl-1,3-dioxolan-5-yl-methoxy)--imino-para-tolylacetonitril gemäss Anspruch 1.

14. N-(1,3-Dioxolan-5-yl-methoxy)-imino-para--tolyl-acetonitril gemäss Anspruch 1.

15. Verfahren zur Herstellung der Oximäther der Formel I gemäss Anspruch 1, dadurch gekennzeichnet, dass man ein Salz eines Oxims der Formel II

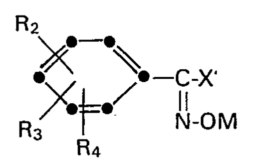

(II)

in welcher M ein Alkalimetall- oder Erdalkalimetallkation darstellt und $R_2$, $R_3$, $R_4$ und X die in Anspruch 1 angegebene Bedeutung haben, mit einem 5-(α-Halogenalkyl oder β-Halogenalkyl)-1,3-dioxolan der Formel III

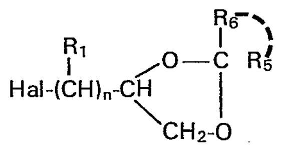

(III)

in welcher Hal ein Halogenatom, insbesondere ein Chloratom oder ein Bromatom, bedeutet und n, $R_1$, $R_5$ und $R_6$ die in Anspruch 1 angegebene Bedeutung haben, umsetzt.

16. Verfahren nach Anspruch 15, dadurch gekennzeichnet, dass man als Salz eines Oxims der Formel II das Natrium- oder Kaliumsalz verwendet.

17. Verfahren nach Anspruch 15, dadurch gekennzeichnet, dass man die Umsetzung des Oxims der Formel II mit dem 5-Halogenalkyl-1,3-dioxolan der Formel III in einem polaren Lösungsmittel durchführt.

18. Mittel zum Schützen von Kulturpflanzen vor Schädigung durch Herbizide, dadurch gekennzeichnet, dass es neben inerten Träger- und Zusatzstoffen als wirksame Komponente einen Oximäther der Formel I gemäss Anspruch 1 enthält.

19. Herbizides Mittel gekennzeichnet durch einen Gehalt an

a) einem herbizid wirksamen Halogenacetanilid oder einem herbizid wirksamen Thiolcarbamat und

b) einem Oximäther der Formel I gemäss Anspruch 1 als Gegenmittel.

20. Herbizides Mittel, gekennzeichnet durch einen Gehalt an

a) einem Halogenacetanilid der Formel VIII

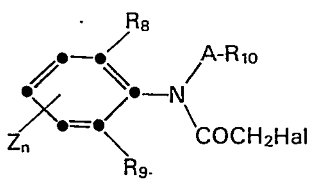

(VIII)

in welcher Hal Halogen, insbesondere Chlor oder Brom, $R_8$ und $R_9$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die Reste Z vorzugsweise in 3-Stellung in bezug auf das Stickstoffatom stehen, n 0 bis 3, A Alkylen, insbesondere Methylen, 1,1- und 1,2-Äthylen, wobei 1,2-Äthylen durch 1-2 niedere Alkylgruppen substituiert sein kann, und $R_{10}$ niederes Alkoxy, Hydroxycarbonyl, Alkoxycarbonyl, Carbamoyl, N-Alkylcarbamoyl, N,N-Dialkylcarbamoyl, Cyano, einen gegebenenfalls substituierten Stickstoff haltigen heterocyclischen Rest, Alkanoyl, gegebenenfalls substituiertes Benzyl, gegebenenfalls substituiertes 1,3,4--Oxadiazol-2-yl, 1,3,4-Thiadiazol-2-yl, 1,3,4-Triazol--3-yl oder 1,3,4-Triazol-1-yl bedeutet, und

b) einem Oximäther der Formel I gemäss Anspruch 1 als Gegenmittel.

21. Herbizides Mittel, gekennzeichnet durch einen Gehalt an

a) einem Thiolcarbamat der Formeln IX und X

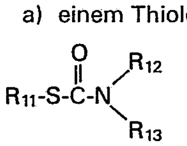 (IX) 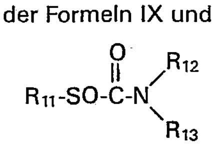 (X)

in welcher $R_{11}$ niederes Alkyl, Alkenyl, Chlorallyl, Dichlorallyl, Trichlorallyl, Benzyl, oder 4-Chlorbenzyl, $R_{12}$ $C_2$-$C_4$ Alkyl und $R_{13}$ $C_2$-$C_4$-Alkyl oder Cyclohexyl bedeutet, wobei die Reste $R_{12}$ und $R_{13}$ zusammen mit dem Stickstoffatom, an das sie gebunden sind, einen Hexahydro-1H-azepin, Dekahydrochinolin- oder 2-Methyldekahydrochinolin-Ring bilden können, und

b) einem Oximäther der Formel I gemäss Anspruch 1 als Gegenmittel.

22. Verwendung der Oximäther der Formel I gemäss Anspruch 1 zum Schützen von Kulturpflanzen gegen die schädigende Wirkung von Herbiziden.

23. Verwendung der Oximäther der Formel I gemäss Anspruch 1 zum Schützen von Getreide-, Mais- und Sorghum-Kulturen vor der phytotoxischen Wirkung von Herbiziden.

24. Verfahren zum Schützen von Kulturpflanzen vor Schäden, die bei der Applikation von Herbiziden auftreten können, dadurch gekennzeichnet, dass man

a) die Anbaufläche für die Pflanze vor oder während der Applikation des Herbizids oder

b) den Samen oder die Stecklinge der Pflanzen oder die Pflanze selbst mit einer wirksamen Menge eines Oximäthers der Formel I gemäss Anspruch 1 behandelt.

25. Die Verwendung der Oximäther der Formel I, Anspruch 1 zur Beeinflussung des Pflanzenwuchses.

26. Die Verwendung der Oximäther der Formel I, Anspruch 1 zur Förderung des Wurzelwachstums von Keimlingen.

## Claims

1. An oxime ether of the formula I

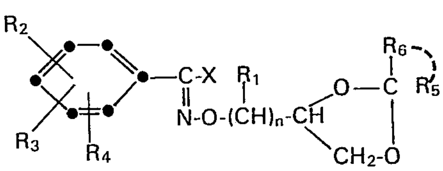

(I)

wherein n is 1 or 2, each of $R_1$ and $R_2$ is hydrogen or $C_1$-$C_4$ alkyl, each of $R_3$ and $R_4$ is hydrogen, halogen, $C_1$-$C_4$ alkyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ haloalkylthio, $C_1$-$C_4$ alkylsulfinyl, $C_1$-$C_4$ alkylsulfonyl, $C_1$-$C_4$ haloalkylsulfinyl, $C_1$-$C_4$ haloalkylsulfonyl or nitro; each of $R_5$ and $R_6$ independently of the other is hydrogen, $C_1$-$C_4$ alkyl, $C_1$-$C_4$ haloalkyl, phenyl or phenyl which is substituted by halogen, $C_1$-$C_4$ alkyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ alkylsulfinyl, $C_1$-$C_4$ alkylsulfonyl, carboxyl, carbamoyl, $C_1$-$C_4$ alkylcarbamoyl, nitro or cyano, or $R_5$ and $R_6$ together are also a 2- to 6-membered alkylene or alkenylene chain which may be substituted by $C_1$-$C_4$ alkyl radicals; X is hydrogen, cyano, nitro, chlorine, $C_1$-$C_4$ alkyl, $C_1$-$C_4$ haloalkyl, $C_3$-$C_6$ cycloalkyl, carboxyl, carbamoyl, $C_1$-$C_4$ alkylcarbonyl, $C_1$-$C_4$ alkoxycarbonyl or $C_1$-$C_4$ alkylcarbamoyl.

2. An oxime ether according to claim 1 of the formula Ia

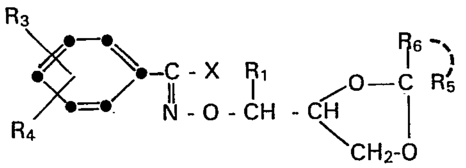 (Ia)

wherein n, $R_3$, $R_5$, $R_6$ and X are as defined in claim 1, $R_4$ is halogen and $R_1$ is hydrogen or methyl.

3. An oxime ether according to claim 1 of the formula Ib

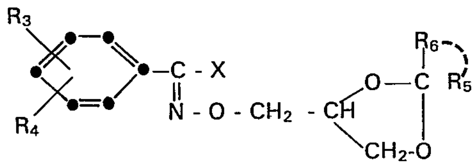 (Ib)

wherein $R_3$, $R_5$ and $R_6$ are as defined in claim 1 and $R_4$ is hydrogen or halogen.

4. An oxime ether according to claim 1 of the formula Ic

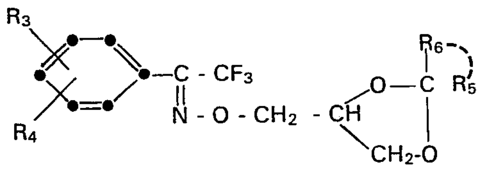 (Ic)

wherein $R_3$, $R_5$ and $R_6$ are as defined in claim 1 and $R_4$ is hydrogen or halogen.

5. N-(1,3-Dioxolan-5-yl-methoxy)imino-para--chlorophenylacetonitrile according to claim 1.

6. N-(2-Methyl-1,3-dioxolan-5-yl-methoxy)imino--para-chlorophenylacetonitrile according to claim 1.

7. Phenyl-1-(1,3-dioxolan-5-yl-methoximino)--2,2,2-trifluorethane according to claim 1.

8. 1-(4-Chlorophenyl)-1-(1,3-dioxolan-5-yl-meth--oximino)-2,2,2-trifluoroethan according to claim 1.

9. N-(2,2-Dimethyl-1,3-dioxolan-5-yl-methoxy)-imino-para-chlorophenylacetonitrile according to claim 1.

10. N-(1,3-Dioxolan-5-yl-methoxy)imino-phenylacetonitrile according to claim 1.

11. N-(2-Methyl-1,3-dioxolan-5-yl-methoxy)imino--ortho-fluorophenylacetonitrile according to claim 1.

12. N-(2-Methyl-1,3-dioxolan-5-yl-methoxy)imino-para-tolylacetonitrile according to claim 1.

13. N-(2,2-Dimethyl-1,3-dioxolan-5-yl-methoxy)-imino-para-tolylacetonitrile according to claim 1.

14. N-(1,3-Dioxolan-5-yl-methoxy)imino-para-tolyl-acetonitrile according to claim 1.

15. A process for the preparation of an oxime ether of the formula I according to claim 1, which process comprises reacting a salt of an oxime of the formula II

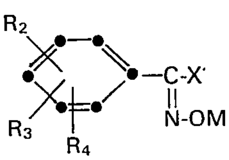 (II)

in which M is an alkali metal cation or an alkaline earth metal cation and $R_2$, $R_3$, $R_4$ and X are as defined in claim 1, with a 5-(α-haloalkyl- or β-haloalkyl)-1,3-dioxolane of the formula III

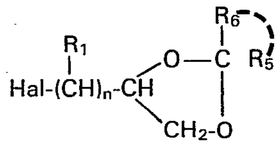 (III)

in which Hal is a halogen atom, preferably a chlorine atom or a bromine atom, and n, $R_1$, $R_5$ and $R_6$ are as defined in claim 1.

16. A process according to claim 15, wherein the sodium or potassium salt is used as salt of an oxime of the formula II.

17. A process according to claim 15, wherein the reaction of the oxime of the formula II with the 5-haloalkyl-1,3-dioxolane of the formula III is carried out in a polar solvent.

18. A composition for protecting cultivated plants from damage caused by herbicides, which contains an oxime ether of the formula I as active component, together with inert carriers and adjuvants.

19. A herbicidal composition which contains

a) a herbicidal haloacetanilide or a herbicidal thiocarbamate, and

b) an oxime ether of the formula I according to claim 1 as antidote.

20. A herbicidal composition which contains

a) a haloacetanilide of the formula VIII

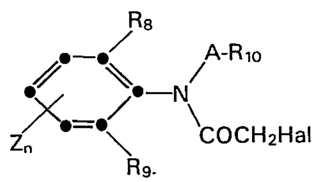 (VIII)

wherein Hal is halogen, preferably chlorine or bromine, each of $R_8$ and $R_9$ independently of the other is hydrogen, halogen, lower alkyl, alkoxy, alkylthio, haloalkyl, alkoxyalkyl or alkylthioalkyl, Z is hydrogen, halogen, lower alkyl, alkoxy, alkylthio, haloalkyl, alkoxyalkyl or alkylthioalkyl, which radicals Z are preferably in the 3-position with respect to the nitrogen atom, n is 0 to 3, A is alkylene, preferably methylene, 1,1-ethylene, and 1,2-ethylene which may be substituted by 1 or 2 lower alkyl groups, and $R_{10}$ is lower alkoxy, hydroxycarbonyl, alkoxycarbonyl, carbamoyl, N-alkylcarbamoyl, N,N-dialkylcarbamoyl, cyano, an unsubstituted or substituted nitrogen-containing heterocyclic radical, alkanoyl, unsubstituted or substituted benzyl, unsubstituted or substituted 1,3,4-oxadiazol-2-yl, 1,3,4-thiadiazol-2-yl, 1,3,4-triazol-3-yl or 1,3,4-triazol-1-yl, and

b) an oxime ether of the formula I according to claim 1 as antidote.

21. A herbicidal composition which contains

a) a thiocarbamate of the formula IX or X

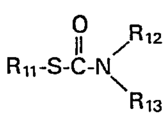 (IX) 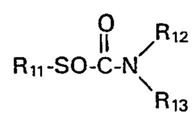 (X)

wherein $R_{11}$ is lower alkyl, alkenyl, chloroallyl, dichloroallyl, trichloroallyl, benzyl or 4-chlorobenzyl, $R_{12}$ is $C_2$-$C_4$ alkyl and $R_{13}$ is $C_2$-$C_4$ alkyl or cyclohexyl, and $R_{12}$ and $R_{13}$ together with the nitrogen atom to which they are attached can form a hexahydro-1H--azepine, decahydroquinoline or 2-methyldecahydroquinoline ring, and

b) an oxime ether of the formula I according to claim 1 as antidote.

22. Use of an oxime ether of the formula I according to claim 1 for protecting cultivated plants from the harmful action of herbicides.

23. Use of an oxime ether of the formula I according to claim 1 for protecting crops of cereals, maize and sorghum from the phytotoxic action of herbicides.

24. A method of protecting cultivated plants from damage caused by the application of herbicides, which method comprises

a) treating the locus of the plant before or during application of the herbicide, or

b) treating the seeds or seedlings of the plant itself with an effective amount of an oxime ether of the formula I according to claim 1.

25. Use of an oxime ether of the formula I according to claim 1 for influencing plant growth.

26. Use of an oxime ether of the formula I according to claim 1 for promoting root growth of seedlings.

**Revendications**

1. Ethers-oximes de formule I

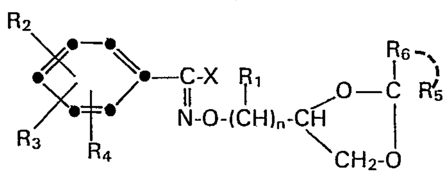

(I)

dans laquelle n est égal à 1 ou 2, $R_1$ et $R_2$ représentent chacun l'hydrogène ou un groupe alkyle en C1-C4, $R_3$ et $R_4$ représentent chacun l'hydrogène, un halogène, un groupe alkyle en C1-C4, halogénoalkyle en C1-C4, alcoxy en C1-C4, alcoxy en C1-C4, halogénoalcoxy en C1-C4, alkylthio en C1-C4, halogénoalkylthio en C1-C4, alkylsulfinyle en C1-C4, alkylsulfonyle en C1-C4, halogénoalkylsulfinyle en C1-C4, halogénoalkylsulfonyle en C1-C4, ou nitro, $R_5$ et $R_6$ représentent chacun, individuellement, l'hydrogène, un groupe alkyle en C1-C4, halogénoalkyle en C1-C4, phényle non substitué ou substitué par des halogènes, alkyle en C1-C4, halogénoalkyle en C1-C4, alcoxy en C1-C4, halogénoalkyle en C1-C4, alkylthio en C1-C4, alkylsulfinyle en C1-C4, alkylsulfonyle en C1-C4, carboxyle, carbamoyle, (alkyle en C1-C4)-carbamoyle, nitro ou cyano, $R_5$ et $R_6$ peuvent également former ensemble une chaîne alkylène ou alcénylène de 2 à 6 chaînons qui peut être substituée par des groupes alkyle en C1-C4, X représente l'hydrogène, un groupe cyano, nitro, le chlore, un groupe alkyle en C1-C4, halogénoalkyle en C1-C4, cycloalkyle en C3-C6, carboxyle, carbamoyle, (alkyle en C1-C4)-carbonyle, (alcoxy en C1-C4)-carbonyle, (alkyle en C1-C4)-carbamoyle.

2. Ethers-oximes selon la revendication 1, de formule Ia

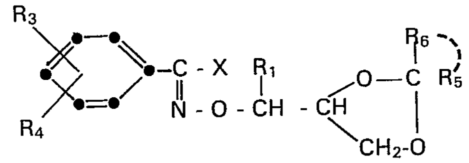

(Ia)

dans laquelle n, $R_3$, $R_5$, $R_6$ et X ont les significations indiquées dans la revendication 1, $R_4$ représente un halogène et $R_1$ l'hydrogène ou un groupe méthyle.

3. Ethers-oximes selon la revendication 1, de formule Ib

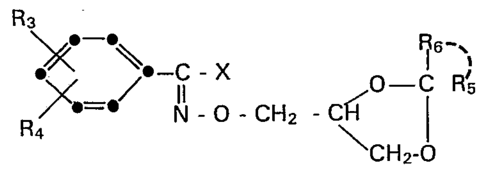

(Ib)

dans laquelle $R_3$, $R_5$ et $R_6$ ont les significations indiquées dans la revendication 1 et $R_4$ représente l'hydrogène ou un halogène.

4. Ether-oximes selon la revendication 1, de formule Ic

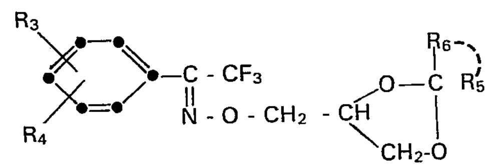

(Ic)

dans laquelle $R_3$, $R_5$ et $R_6$ ont les significations indiquées dans la revendication 1 et $R_4$ représente l'hydrogène ou un halogène.

5. Le N-(1,3-dioxolanne-5-yl-méthoxy)-imino-para-chlorophénylacétonitrile selon la revendication 1.

6. Le N-(2-méthyl-1,3-dioxolanne-5-yl-méthoxy)--imino-para-chlorophénylacétonitrile selon la revendication 1.

7. Le 1-phényl-1-(1,3-dioxolanne-5-yl-méthoximino)-2,2,2-trifluoréthane selon la revendication 1.

8. Le 1-(4-chlorophényl)-1-(1,3-dioxolanne-5-yl--méthoximino)-2,2,2-trifluoroéthane selon la revendication 1.

9. Le N-(2,2-diméthyl-1,3-dioxolanne-5-yl-méthoxy)-imino-para-chlorophénylacétonitrile selon la revendication 1.

10. Le N-(1,3-dioxolanne-5-yl-méthoxy)-imino--phénylacétonitrile selon la revendication 1.

11. Le N-(2-méthyl-1,3-dioxolanne-5-yl-méthoxy)--imino-ortho-fluorophénylacétonitrile selon la revendication 7.

12. Le N-(2-méthyl-1,3-dioxolanne-5-yl-méthoxy)--imino-para-tolylacétonitrile selon la revendication 1.

13. Le N-(2,2-diméthyl-1,3-dioxolanne-5-yl-méthoxy)-imino-para-tolylacétonitrile selon la revendication 1.

14. Le N-(1,3-dioxolanne-5-yl-méthoxy)-imino--para-tolyl-acétonitrile selon la revendication 1.

15. Procédé de préparation des éthers-oximes de formule I selon la revendication 1, caractérisé en ce que l'on fait réagir un sel d'une oxime de formule II

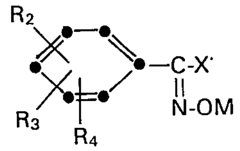 (II)

dans laquelle M représente un cation de métal alcalin ou alcalino-terreux et $R_2$, $R_3$, $R_4$ et X ont les significations indiquées dans la revendication 1, avec un 5-(alpha-halogénoalkyl ou bêta-halogénoalkyl)-1,3-dioxolanne de formule III

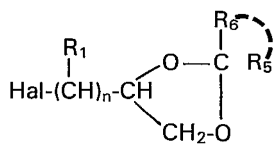 (III)

dans laquelle Hal représente un atome d'halogène, en particulier un atome de chlore ou un atome de brome, et n, $R_1$, $R_5$ et $R_6$ ont les significations indiquées dans la revendication 1.

16. Procédé selon la revendication 15, caractérisé en ce que le sel d'une oxime de formule II est le sel de sodium ou de potassium.

17. Procédé selon la revendication 15, caractérisé en ce que la réaction entre l'oxime de formule II et le 5-halogénoalkyl-1,3-dioxolanne de formule III est effectuée dans un solvant polaire.

18. Produit pour protéger les végétaux cultivés contre les dégâts provoqués par des herbicides, caractérisé en ce qu'il contient en tant que composant actif, avec des véhicules et additifs inertes, un éther-oxime de formule I selon la revendication 1.

19. Produit herbicide caractérisé en ce qu'il contient:

a) un halogénoacétanilide herbicide actif ou un thiolcarbamate herbicide actif, et

b) un éther-oxime de formule I selon la revendication 1, en tant qu'antidote.

20. Produit herbicide caractérisé en ce qu'il contient:

a) un halogénoacétanilide de formule VIII

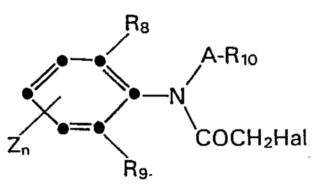 (VIII)

dans laquelle Hal représente un halogène, en particulier le chlore ou le brome, $R_8$ et $R_9$ représentent chacun, indépendamment l'un de l'autre, l'hydrogène, un halogène, un groupe alkyle, alcoxy, alkylthio, halogénoalkyle, alcoxyalkyle ou alkylthioalkyle inférieur, Z représente l'hydrogène, un halogène, un groupe alkyle, alcoxy, alkylthio, halogénoalkyle, alcoxyalkyle ou alkylthioalkyle inférieur, les restes Z étant de préférence en position 3 par rapport à l'atome d'azote, n est un nombre de 0 à 3, A représente un groupe alkylène, en particulier méthylène, 1,1- et 1,2-éthylène, le groupe 1,2-éthylène pouvant être substitué par un ou deux groupes alkyle inférieur, et $R_{10}$ représente un groupe alcoxy inférieur, hydroxycarbonyle, alcoxycarbonyle, carbamoyle, N-alkylcarbamoyle, N,N-dialkylcarbamoyle, cyano, un reste hétérocyclique azoté éventuellement substitué, un groupe alcanoyle, benzyle éventuellement substitué, 1,3,4-oxadiazole-2-yle, 1,3,4-thiadiazole-2-yle, 1,3,4-triazole-3-yle ou 1,3,4-triazole-1-yle éventuellement substitué, et

b) un éther-oxime de formule I selon la revendication 1, in tant qu'antidote.

21. Produit herbicide caractérisé en ce qu'il contient:

a) un thiolcarbamate de formules IX et X

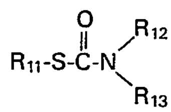 (IX) 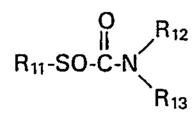 (X)

dans lesquelles $R_{11}$ représente un groupe alkyle inférieur, alcényle, chlorallyle, dichlorallyle, trichlorallyle, benzyle ou 4-chlorobenzyle, $R_{12}$ représente un groupe alkyle en C2-C4 et $R_{13}$ un groupe alkyle en C2-C4 ou cyclohexyle, les restes $R_{12}$ et $R_{13}$ pouvant former avec l'atome d'azote auquel ils sont reliés un cycle hexahydro-1H-azépine, décahydroquinoléine ou 2-méthyldécahydroquinoléine, et

b) un éther-oxime de formule I selon la revendication 1, en tant qu'antidote.

22. Utilisation des éthers-oximes de formule I selon la revendication 1 dans la protection des végétaux cultivés contre les effets nocifs des herbicides.

23. Utilisation des éthers-oximes de formule I selon la revendication 1 dans la protection des cultures de céréales, de maïs et de sorgho, contre les effets phytotoxiques des herbicides.

24. Procédé pour protéger les végétaux cultivés contre les dégâts susceptibles de se produire à l'application des herbicides, caractérisé en ce que:

a) on traite la surface de culture des végétaux avant ou durant l'application de l'herbicide, ou bien

b) on traite les semences ou les pousses des végétaux ou les végétaux eux-mêmes par une quantité efficace d'un éther-oxime de formule I selon la revendication 1.

25. L'utilisation des éthers-oximes de formule I, revendication 1, pour agir sur la croissance des végétaux.

26. L'utilisation des éthers-oximes de formule I, revendication 1, pour activer la croissance des racines de plants.